# EUROPÄISCHE PATENTSCHRIFT

(11) **EP 2 861 277 B1**
(45) Veröffentlichungstag und Bekanntmachung des Hinweises auf die Patenterteilung: **12.05.2021**
(21) Anmeldenummer: 13729947.5
(22) Anmeldetag: 18.06.2013
(51) Int. Cl.: A61M 5/158

(54) **PORTKANÜLENSYSTEM ZUM PUNKTIEREN VON PORTKATHETERN**
PORT CANNULA SYSTEM WITH FOLDABLE SUPPORT FOR PUNCTURING PORT CATHETERS
CANULES D'ACCÈS AVEC BASE ARTICULÉE DESTINÉS À L'IMPLANTAION DE CATHÉTERS D'ACCÈS

(30) Priorität: 18.06.2012 EP 12172480; 18.06.2012 US 201261660986 P
(43) Veröffentlichungstag der Anmeldung: 22.04.2015
(73) Patentinhaber: Fresenius Kabi Deutschland GmbH, 61352 Bad Homburg (DE)
(72) Erfinder: PAPIOREK, Martina, 65597 Hünfelden (DE)
(74) Vertreter: Fresenius Kabi Deutschland GmbH
(86) Internationale Anmeldenummer: PCT/EP2013/062598
(87) Internationale Veröffentlichungsnummer: WO 2013/189918

(56) Entgegenhaltungen:
- EP-A1- 0 769 302
- EP-A1- 2 404 547
- WO-A1-99/15221
- WO-A1-2009/055739
- WO-A1-2010/142641
- DE-A1-102008 052 787
- US-A- 4 769 010
- US-A- 5 951 522
- US-A1- 2009 299 302

## Beschreibung

### Beschreibung der Erfindung

Die vorliegende Erfindung bezieht sich auf Portkanülensysteme zum Punktieren von implantierbaren bzw. implantierten Portkathetern.

### Hintergrund der Erfindung

Portkanülensysteme sind im Stand der Technik zahlreich vorhanden und werden genutzt, um eine Verbindung zu einem im menschlichen oder tierischen Körper implantierten Port herzustellen. Solche Ports werden implantiert, um einfacher und zuverlässiger Substanzen in den menschlichen Körper, insbesondere in seine zentralen Gefäße, einzubringen.

Zur Verbindung mit einem Port weist das Portkanülensystem eine Kanüle mit einer Kanülenspitze auf. Eine solche Kanülenspitze ist gefährlich und wird daher im Auslieferungszustand in der Regel durch einen Kanülenschutz, z.B. in Form einer auf die Kanüle steckbaren Hülse, umschlossen. Der Kanülenschutz wird erst kurz vor Applizierung der Portkanüle entfernt. Die Kanüle ist zu diesem Zeitpunkt steril und stellt zwar ein Verletzungsrisiko, aber kein Infektionsrisiko für den Anwender dar.

Wird das Portkanülensystem hingegen aus dem Patienten bzw. aus dem Port des Patienten entfernt, ist die Kanüle nicht mehr steril und stellt somit nicht nur ein Verletzungsrisiko, sondern auch ein hohes Infektionsrisiko, nicht nur für das medizinische Personal, sondern auch für das Personal der Abfallwirtschaft dar. Aus diesem Grund ist es wünschenswert, Portkanülensysteme mit einer Nadelstichschutzvorrichtung auszurüsten, welche insbesondere beim und nach Entfernen des Portkanülensystem nach der Anwendung vor Stichverletzungen schützt.

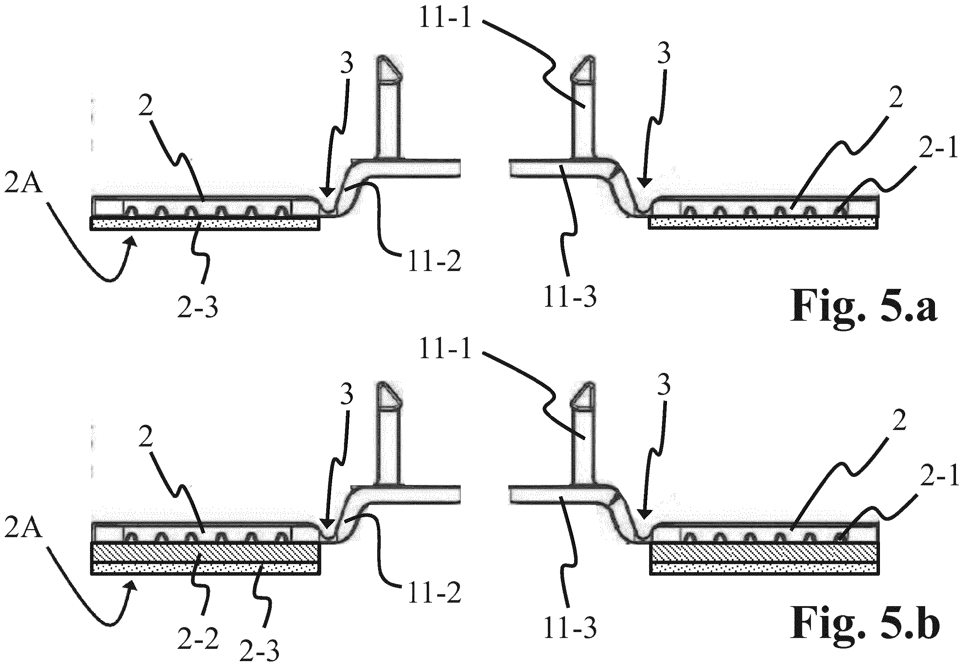

Ein Portkanülensystem mit einer wirksamen Nadelstichschutzvorrichtung ist zum Beispiel in dem Dokument WO 2010/142641 A1 beschrieben. Das Portkanülensystem weist folgende Bestandteile auf: eine Auflage zur Auflage der Portkanüle am Patienten, eine Zuführung, eine Kanüle mit einer Spitze, wobei die Kanüle in mindestens zwei Positionen, eine Punktionsstellung und eine zurückgezogene Schutzstellung, gebracht werden kann, und einen Sicherungsabschnitt, der die Spitze der Kanüle mindestens in der zurückgezogenen Stellung abschirmt und so eine Nadelstichschutzvorrichtung bildet. Der Sicherungsabschnitt wird bereitgestellt durch Teleskopsegmente, die in der Punktionsstellung weitestgehend ineinander geschoben und in der zurückgezogenen Stellung weiter auseinander gezogen sind (siehe dazu auch die Figuren 5.a und 5.b des vorliegenden Anmeldung). Die an ein Portkanülensystem gestellten Sicherheitsanforderungen werden durch das in der WO 2010/142641 A1 beschriebene Portkanülensystem sehr gut erfüllt. Das Portkanülensystem beinhaltet ein großflächiges, die Kanülenhalterung vollständig umfassendes, integriertes Pflaster und ein integriertes Nivellierungselement zur großflächigen Befestigung auf der Haut eines Patienten. Das dort beschriebene Portkanülensystem ist insbesondere geeignet für Anwender, welche nach der Punktion eine schnelle und einfache Befestigung des Portkanülensystems wünschen und für deren Patienten ein täglicher Verbandswechsel nicht notwendig ist.

### Allgemeine Beschreibung der Erfindung

Vor dem vorstehend geschilderten Hintergrund liegt der vorliegenden Erfindung die Aufgabe zugrunde, ein alternatives Portkanülensystem bereitzustellen. Das Portkanülensystem soll insbesondere einfacher herstellbar sein.

Gelöst werden die Aufgaben bereits durch das Portkanülensystem gemäß dem unabhängigen Patentanspruch 1. Vorteilhafte Ausführungsformen des erfindungsgemäßen Portkanülensystems sind Gegenstand der abhängigen Unteransprüche.

Die vorliegende Erfindung wird beschrieben durch ein Portkanülensystem, welches die folgenden Komponenten umfasst: eine Kanülenhalterung, eine an der Kanülenhalterung befestigte Kanüle mit einer Kanülenspitze, die wenigstens in eine Punktionsstellung und eine Sicherungsstellung überführbar ist, einen Sicherungsabschnitt, der die Kanülenspitze in der Sicherungsstellung zumindest abschnittsweise umgibt, eine an der Kanüle angeschlossene Zuführung sowie wenigstens eine mit der Kanülenhalterung verbundene Auflage zum Auflegen des Portkanülensystems auf einer Haut eines Patienten, wobei die wenigstens eine Auflage mittels eines Scharniers mit der Kanülenhalterung verbunden ist.

Die Auflage stellt die Auflagefläche für das Portkanülensystem auf der Haut des Patienten bereit. Sie ist eine Art Träger für das Portkanülensystem. Zum Beispiel besitzt die Auflage eine Auflagefläche von etwa 5 mm bis 30 mm mal 5 mm bis 30 mm, vorzugsweise von 10 mm bis 20 mm mal 10 mm bis 20 mm. Die Dicke der Auflage beträgt etwa 0,5 bis 4mm, vorzugsweise 1 mm bis 2 mm. Die Auflage ist über das Scharnier beweglich an dem Portkanülensystem bzw. der Kanülenhalterung befestigt. Das Scharnier ermöglicht einen Höhenausgleich, wenn zum Beispiel die Haut des Patienten in dem Applikationsgebiet nicht eben ist oder wenn sich der Patient bewegt. Die Auflage stellt dadurch, in Verbindung mit dem Scharnier, eine Art Nivellierungselement dar.

Die Auflage stellt keine Vorrichtung zum Greifen des Portkanülensystems dar, mittels der das Portkanülensystem gegriffen und von der Haut eines Patienten entfernt werden kann. Daher ist die Auflage im bestimmungsgemäßen Gebrauch auch nicht nach oben, zum Beispiel um mindestens 90° nach oben oder relativ zu einer Horizontalen, klappbar oder schwenkbar. Der Schwenkbereich der Auflage um eine durch das Scharnier definierte Achse ist kleiner. Denn das Scharnier soll im Wesentlichen lediglich einen Höhenausgleich ermöglichen, um ein definiertes Aufliegen des Portkanülensystems auf einer beispielsweise unebenen Haut zu ermöglichen. In einer Ausführungsform ist die Auflage im bestimmungsgemäßen Gebrauch um eine durch das Scharnier definierte Achse in einem Winkelbereich α von 0° < α ≤ 270° schwenkbar. Der Winkel α ist definiert durch den Winkel, welcher zwischen der Längsachse des Portkanülensystems und der Auflage, insbesondere der Oberseite der Auflage, eingeschlossen wird.

In einer bevorzugten Ausführungsform der Erfindung ist das Scharnier ein, vorzugsweise lineares, Filmscharnier. Eine solche Ausgestaltung ist einfach herstellbar. Insbesondere kann der beschränkte Schwenkbereich der Auflage durch die Abmessungen des Filmscharniers eingestellt werden.

Insbesondere besitzt das Faltscharnier an seiner dünnsten Stelle eine Dicke, die um einen Faktor von kleiner als 1/2, vorzugsweise von kleiner als 1/3, reduziert gegenüber der Dicke der Auflage ist. In einer Ausgestaltung ha das Faltscharnier an seiner dünnsten Stelle eine Dicke in einem Bereich von 0,1 mm bis 1 mm, vorzugsweise von 0,2 mm bis 0,6 mm. Der Kurvenradius der das Faltenscharnier bildenden Aussparung oder Verdünnung liegt in einem Bereich von 0,1 mm bis 1 mm, vorzugsweise von 0,3 mm bis 0,7 mm.

Insbesondere wird die Auflage durch wenigstens zwei Platten bereitgestellt, die sich seitlich von der Kanülenhalterung nach außen erstrecken. Die zwei Platten sind vorzugsweise gegenüberliegend angeordnet. Die zwei Platten stellen in dieser Ausgestaltung eine Art Flügelanordnung dar. Die zwei Platten oder Flügel sind jeweils über ein Scharnier mit der Kanülenhalterung verbunden.

Die Kanülenhalterung trägt unmittelbar oder mittelbar die Kanüle. Vorzugsweise ist sie mehrteilig aufgebaut. Erfindungsgemäß umfasst sie ein mit der Auflage verbundenes unteres Halterungssegment, welches eine Stufe bereitstellt, so dass das untere Halterungssegment und somit auch die Kanülenhalterung insgesamt in einem auf der Haut des Patienten aufgelegten Zustand des Portkanülensystems von der Haut des Patienten beabstandet ist. Es wird ein Zwischenraum zwischen der Unterseite der Kanülenhalterung und der Haut des Patienten gebildet. Das untere Halterungssegment der Kanülenhalterung bildet zur Seite hin eine Stufe aus, welche an ihrer untersten Stelle an die Auflage angrenzt. Das Scharnier befindet sich zwischen der Stufe und der Auflage. Das Scharnier verbindet die Stufe des unteren Halterungssegments mit der Auflage. Der durch die Stufe bereitgestellte Zwischenraum unter dem System ermöglicht eine bessere Beobachtung der Einstichstelle. Die bessere Beobachtung wird im Allgemeinen gewährleistet durch einen täglichen Verbandswechsel, zu dessen Zweck eine Kompresse unter das Portkanülensystem gelegt wird. An dieser Kompresse können Sekret-Absonderungen beim Verbandswechsel erkannt werden.

Insbesondere sind die Auflage und/oder die Kanülenhalterung mittels Spritzguss gefertigt. Ein bevorzugtes Material für die Auflage und/oder die Kanülenhalterung ist Polypropylen. Insbesondere in der Ausgestaltung, in der das Scharnier als ein Filmscharnier ausgebildet ist, sind zumindest das untere Halterungssegment der Kanülenhalterung und die Auflage einteilig. Es entsteht dadurch ein einzelnes Teil, das in einem Fertigungsschritt und somit kostengünstig produziert werden kann.

In einer möglichen Ausgestaltung der Auflage ist an der Unterseite der Auflage zumindest abschnittsweise eine Vielzahl an Rillen eingebracht. Diese ermöglichen eine hohe Flexibilität der Auflage. Ferner wird eine Luftzirkulation und somit eine Reduzierung der Feuchtigkeitsentwicklung durch Hautkontakt im Falle einer partiellen Hautpolsterauflage-Geometrie sichergestellt.

In einer weiteren Ausführungsform ist an der Unterseite der Auflage zumindest abschnittsweise ein Polster oder Auflagepolster angeordnet. Dadurch kann der Tragekomfort des Portkanülensystems für den Patienten erhöht werden. Mögliche Materialen für das Polster oder Auflagepolster sind Viskose-Polypropylen und/oder eine Polyethersulfone/Polyethylen-Mischung und/oder eine Polyethylen/Polyurethan-Mischung (insbesondere als Wundkissen) und/oder ein Polyestervlies (insbesondere als Wund- und/oder Hautauflage) und/oder eine vorzugsweise transparente Polyethylen-Folie (insbesondere als Hautauflage) und/oder ein Polyethylen-Schaum (insbesondere als Hautauflagepolster), vorzugsweise jeweils kombiniert und/oder beschichtet mit einem Kleber, beispielsweise einem Polyacrylatkleber. Die Auflagefläche des Polsters kann im Wesentlichen der Auflagefläche der erfindungsgemäßen Auflage entsprechen. Das Polster besitzt beispielsweise eine Dicke in einem Bereich von 0,1 mm bis 3 mm, vorzugsweise von 0,5 mm bis 1,5 mm.

In einer alternativen oder ergänzenden Ausführungsform ist eine Unterseite der Auflage und/oder des Polsters zumindest abschnittsweise mit einer Schicht eines Klebstoffs zum lösbaren Verbinden der Auflage mit der Haut des Patienten bedeckt. Dadurch wird ein einfaches und sicheres Befestigen des Systems auf der Haut eines Patienten ermöglicht. Um ein Austrocknen des Klebstoffs zu verhindern, ist der Klebstoff im Allgemeinen mit einer abziehbaren Folie bedeckt. Ergänzend kann das Portkanülensystem noch mit einem bzw. zwei separaten Klebestreifen befestigt werden.

Wie es bereits vorstehend ausgeführt ist, ist die Kanüle in mindestens zwei Positionen, eine Punktionsstellung und eine, vorzugsweise zurückgezogene, Sicherungsstellung, bringbar. In der Punktionsstellung liegt die Kanüle oder zumindest die Kanülenspitze frei, um in einen implantierten Port einzustechen. In der Punktionsstellung gibt der Sicherungsabschnitt wenigstens einen Teil der Kanüle oder zumindest die Kanülenspitze frei. In der Sicherungsstellung ist die Kanüle oder zumindest die Kanülenspitze in eine Stellung zurückgezogen oder in einer Stellung positioniert, in der sie abgeschirmt ist und zum Beispiel einen Anwender nicht verletzen kann. Dazu ist der Sicherungsabschnitt vorgesehen, der die Kanülenspitze mindestens in der, vorzugsweise zurückgezogenen, Stellung umgibt oder abschirmt und so eine Nadelstichschutzvorrichtung bildet. In der Sicherungsstellung ist die Kanüle oder zumindest die Kanülenspitze in einem durch den Sicherungsabschnitt bereitgestellten Innenraum, vorzugsweise zentrisch, positioniert. Der Sicherungsabschnitt ist mit der Kanülenhalterung verbunden. Insbesondere ist der Sicherungsabschnitt zum Bereitstellen der Sicherungsstellung, vorzugsweise entlang seiner Längsachse, ausziehbar. Der Sicherungsabschnitt kann auch als Nadelstichschutzvorrichtung bezeichnet werden.

In einer Ausgestaltung der Kanülenhalterung weist sie wenigstens ein mit dem unteren Halterungssegment verbundenes mittleres Halterungssegment und ein mit dem mittleren Halterungssegment verbundenes oberes Halterungssegment auf. Weiterhin kann die Kanülenhalterung noch ein Griffstück zum Bedienen des Portkanülensystems aufweisen, welches vorzugsweise mit dem oberen Halterungssegment verbunden ist. Insbesondere ist die Zuführung an der Kanülenhalterung, insbesondere an dem Griffstück, befestigt.

In einer bevorzugten Ausführungsform der vorliegenden Erfindung sind das obere Halterungssegment, das wenigstens eine mittlere Halterungssegment und/oder das untere Halterungssegment Teleskopsegmente, die gegeneinander beweglich und untereinander geführt ausgebildet sind, wobei die Teleskopsegmente in der Punktionsstellung zumindest abschnittsweise ineinander geschoben sind und in der zurückgezogenen Schutzstellung weiter auseinander gezogen sind. In der Sicherungsstellung wird zumindest die Kanülenhalterung durch die genannten Teleskopsegmente abgeschirmt. Die Teleskopsegmente bilden in dieser Variante der Erfindung den Sicherungsabschnitt. Die Kanüle und die Kanülenspitze sind in dem durch die Teleskopsegmente bereitgestellten Innenraum angeordnet.

In einer Ausführungsform wird bzw. werden die Kanülenhalterung, die Auflage, die Zuführung, das untere Halterungssegment, das mittlere Halterungssegment, das obere Halterungssegment, das Griffstück, das Polster, der Kleber für das Polster und/oder der Kleber zum Verbinden mit der Haut durch ein transparentes Material oder im Wesentlichen transparentes Material, insbesondere einen transparenten Kunststoff, bereitgestellt. Vorzugsweise werden alle Komponenten des Portkanülensystems durch ein transparentes Material gebildet. Beispielsweise kann beim Auflegen einer Wand einer Komponente auf ein weißes, mit schwarzen Ziffern bedrucktes Blatt der Zifferndruck durch die Wand hindurch erkannt werden. Dadurch wird eine verbesserte Beobachtung und Überwachung der Einstichstelle und ihrer Umgebung gewährleistet.

Weiterhin liegt im Bereich der vorliegenden Erfindung auch ein Set umfassend ein Portkanülensystem mit den vorstehend beschriebenen Eigenschaften, einen Klebestreifen zum Befestigen des Portkanülensystems auf der Haut eines Patienten, eine Kompresse zum Positionieren zwischen dem Portkanülensystem und der Haut eines Patienten, eine auf die Kanüle aufsetzbare oder aufgesetzte Nadelschutzkappe, einen mit der Zuführung verbundenen oder verbindbaren Schlauch, eine Schlauchklemme und/oder einen mit dem Schlauch verbundenen oder verbindbaren Konnektor, insbesondere einen weiblichen Luer-Lock-Konnektor.

Die vorliegende Erfindung wird anhand der nachfolgenden Ausführungsbeispiele im Einzelnen erläutert. Hierzu wird auf die beigefügten Zeichnungen Bezug genommen. Die gleichen Bezugszeichen in den einzelnen Zeichnungen beziehen sich auf die gleichen Teile.
Fig. 1.a und 1.b zeigen eine schematische Darstellung des Portkanülensystems (ohne Auflage) in seiner Punktionsstellung (Fig. 1.a) und seiner zurückgezogenen Schutzstellung (Fig. 1.b) in einem Querschnitt.
Fig. 2 zeigt eine schematische Darstellung eines erfindungsgemäßen Portkanülensystems (mit Kanüle) in einer perspektivischen Darstellung auf seine Oberseite.
Fig. 3.a bis 3.c zeigen schematische Darstellungen eines erfindungsgemäßen Portkanülensystems (ohne Kanüle) in zwei perspektivischen Darstellungen (Fig. 3.a und 3.b) und in einer Aufsicht (Fig. 3.c).
Fig. 4.a bis 4.c zeigen verschiedene schematische Darstellungen einer 1-teilig ausgebildeten Kanülenhalterung mit zwei Auflagen in einer perspektivischen Ansicht auf die Oberseite (Fig. 4.a), auf die Unterseite (Fig. 4.b) und in einem Querschnitt (Fig. 4.c).
Fig. 5.a und 5.b zeigen schematische Darstellungen zweier Ausführungsformen der Kanülenhalterung aus der Figur 4.c mit einer auf der Unterseite der Auflagen aufgebrachten Klebeschicht (Fig. 5.a) und einer Polsterung mit Klebeschicht (Fig. 5.b).

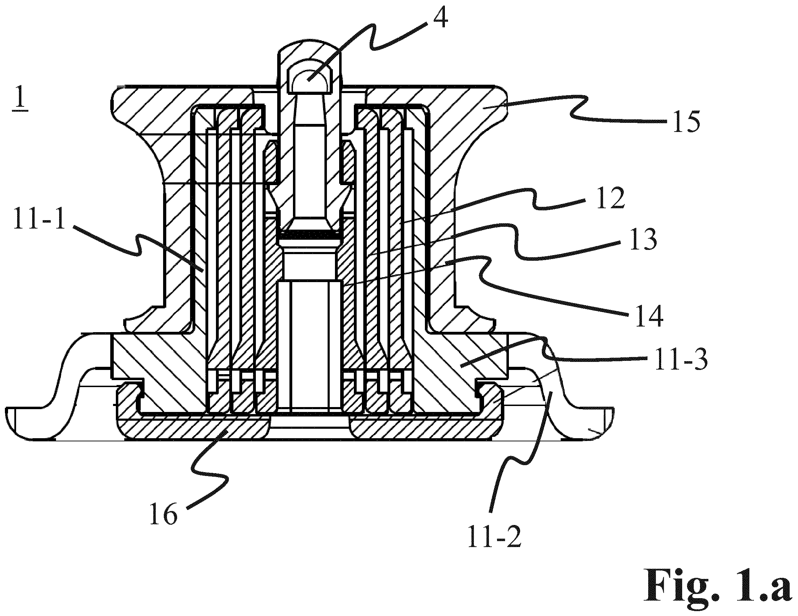

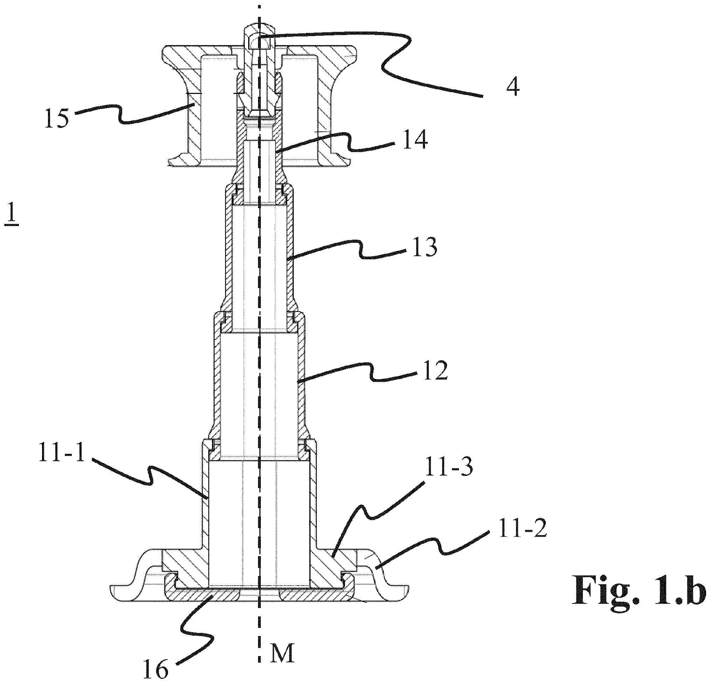

Die vorliegende Erfindung wird illustriert am Beispiel eines Portkanülensystems 1, das eine Nadelstichschutzvorrichtung beinhaltet, wie sie in dem Dokument WO 2010/142641 A1 beschrieben ist. Deren Prinzip basiert im Wesentlichen darauf, dass die Kanüle 5 in mindestens zwei Stellungen, eine Punktionsstellung (siehe Figur 1.a) und eine zurückgezogene Schutzstellung (siehe Figur 1.b), in welcher die Kanülenspitze der Kanüle 5 durch einen Sicherungsabschnitt abgeschirmt ist, überführbar ist. Der Sicherungsabschnitt wird in dieser Variante bereitgestellt durch Teleskopsegmente 11-1, 12, 13 und 14, welche in der Punktionsstellung weitestgehend ineinander geschoben sind und in der zurückgezogenen Stellung weiter auseinander gezogen sind. Figur 1.a illustriert das Portkanülensystem 1 in der Punktionsstellung, in der die Teleskopsegmente 11-1, 12, 13, 14 ineinander geschoben sind. Figur 1.b illustriert dagegen das Portkanülensystem 1 in der zurückgezogenen Schutzstellung, in welcher die Teleskopsegmente 11-1, 12, 13 und 14 auseinander gezogen sind. Die Kanülenspitze der Kanüle 5 wird durch die Teleskopsegmente 11-1, 12, 13 und 14 abgeschirmt. Beim Herausziehen der Kanüle 5 wird das Portkanülensystem 1 durch einen Anwender zum Beispiel über die zwei Auflagen 2 auf die Haut des Patienten gedrückt, das obere Griffstück 15 gepackt und nach oben gezogen.

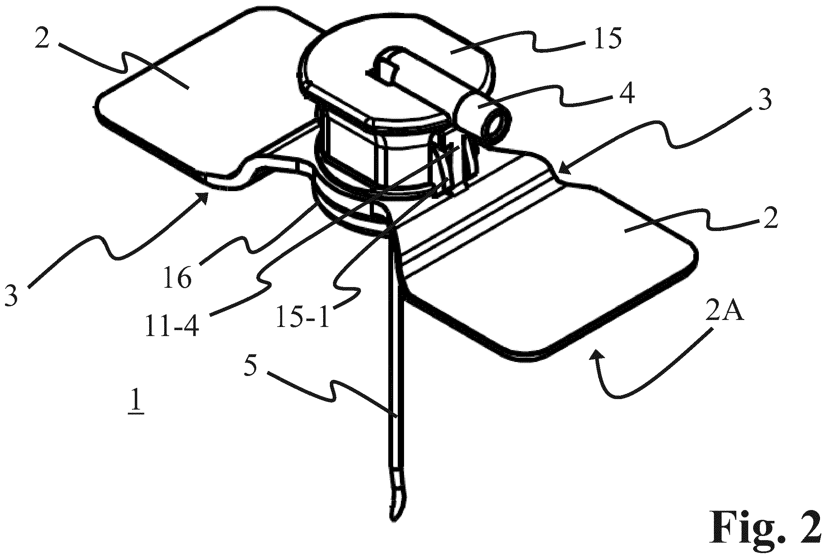

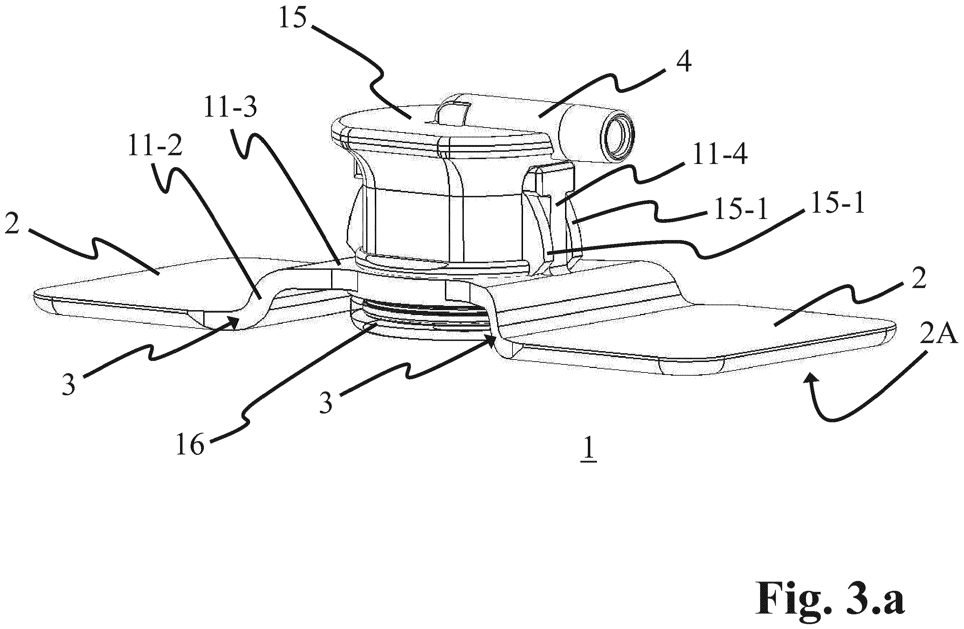

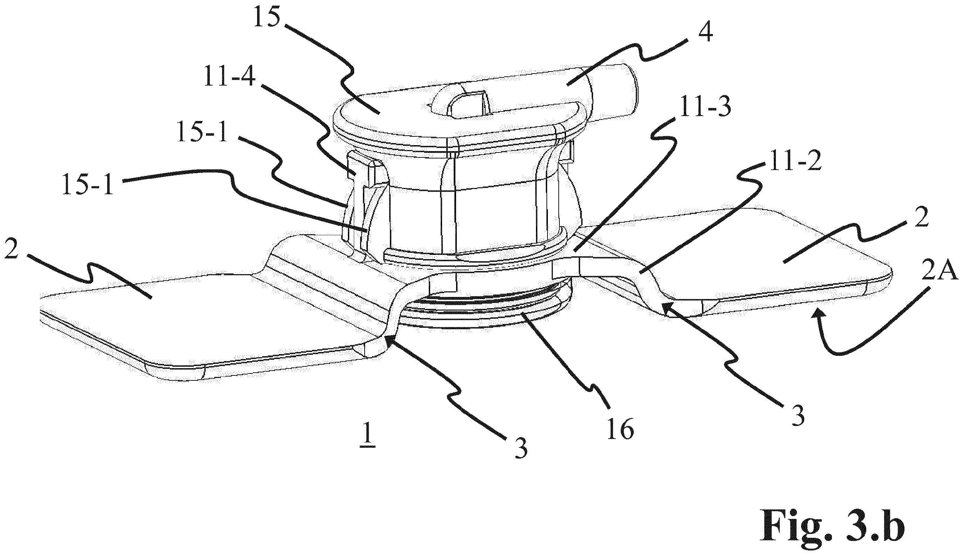

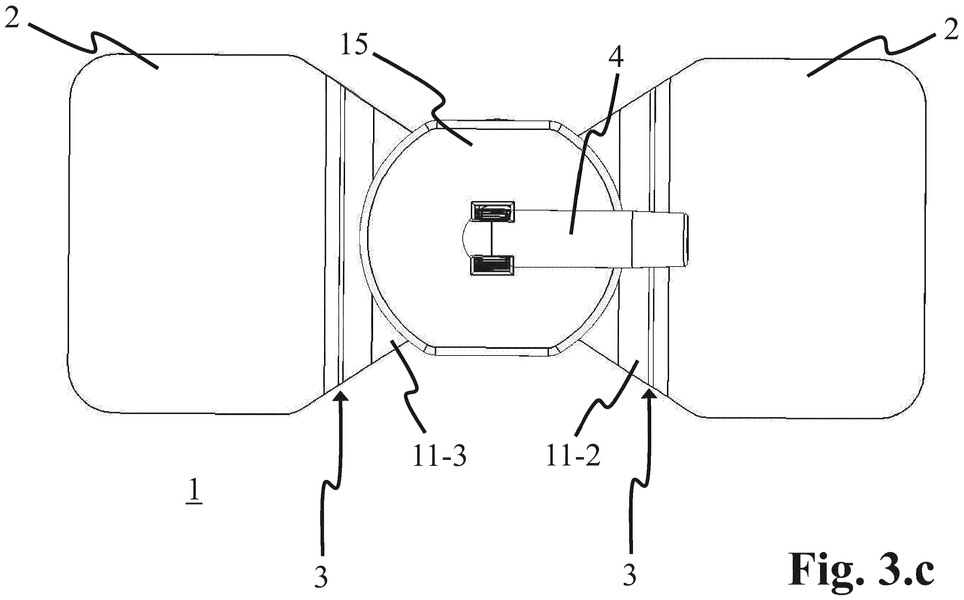

Die Figuren 2 bis 3.c illustrieren das erfindungsgemäße Portkanülensystem 1 aus verschiedenen Perspektiven. Das Portkanülensystem 1 wird gebildet durch die folgenden Komponenten: Eine Kanüle 5, die durch eine Kanülenhalterung 10 getragen wird. Die Kanülenhalterung 10 wird hier gebildet durch ein Griffstück 15, ein mit dem Griffstück 15 verbundenes oberes Halterungssegment 14, ein erstes mittleres Halterungssegment 13, ein zweites mittleres Halterungssegment 12 und ein unteres Halterungssegment 11. Die vier Halterungssegmente 11, 12, 13 und 14 sind teleskopartig gegeneinander bzw. ineinander verschiebbar. Das obere Halterungssegment 14 und die beiden mittleren Halterungssegmente 12 und 13 können daher als Teleskopsegmente bezeichnet werden. Das untere Halterungssegment 11 umfasst hier eine untere Platte 11-3, zwei seitlich an der unteren Platte 11-3 angeordnete Stufen 11-2, ein unteres feststehendes Teleskopsegment 11-1 und zwei Klipslaschen 11-4. Die zwei Klipslaschen 11-4 wirken zusammen mit zwei an der Außenseite des Griffstücks 15 angebrachten Gleitrippen 15-1. Die Klipslaschen 11-4 greifen in die Gleitrippen 15-1 ein und stellen eine Rasterverbindung für die Punktionsstellung bereit. Um ein "Herausfallen" der Teleskopsegmente 12, 13 und 14 zu verhindern, besitzt das Portkanülensystem 1 noch eine mit dem unteren Halterungssegment 11 verbindbare Bodenplatte 16 mit einer Durchgangsöffnung für die Kanüle 5.

An dem unteren Halterungssegment 11, genauer an der unteren Platte 11-3 des unteren Halterungssegments 11, sind zum Beispiel zwei Auflagen 2 zum Auflegen auf der Haut eines Patienten angeordnet oder befestigt. Die zwei Auflagen 2 sind beweglich angeordnet. Sie stellen Flügel dar, die am unteren Halterungssegment 11 der Kanülenhalterung 10 angeordnet oder befestigt sind. Die zwei Auflagen 2 sind mit der Kanülenhalterung 10 mittels zweiter Folienscharniere 3 verbunden.

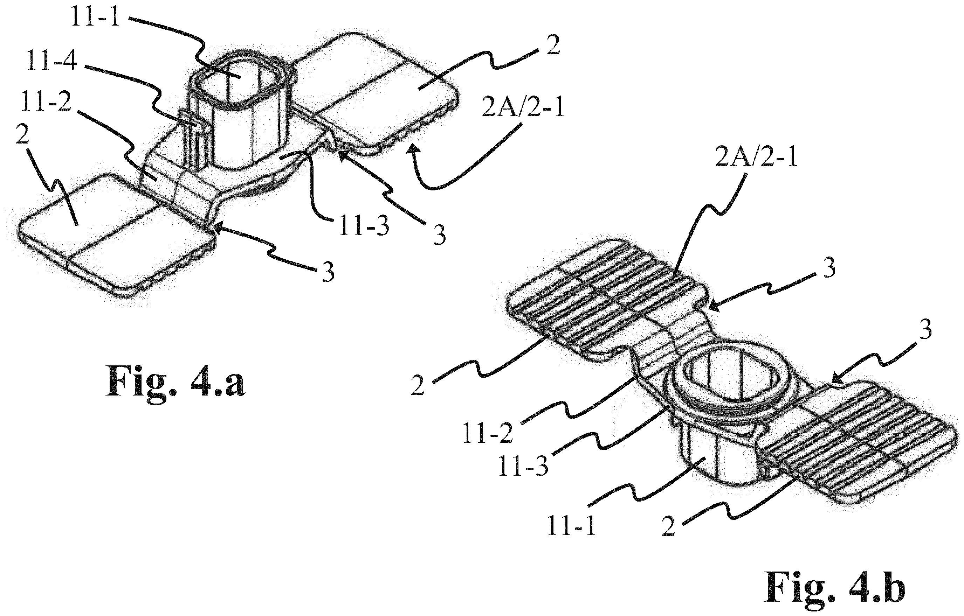

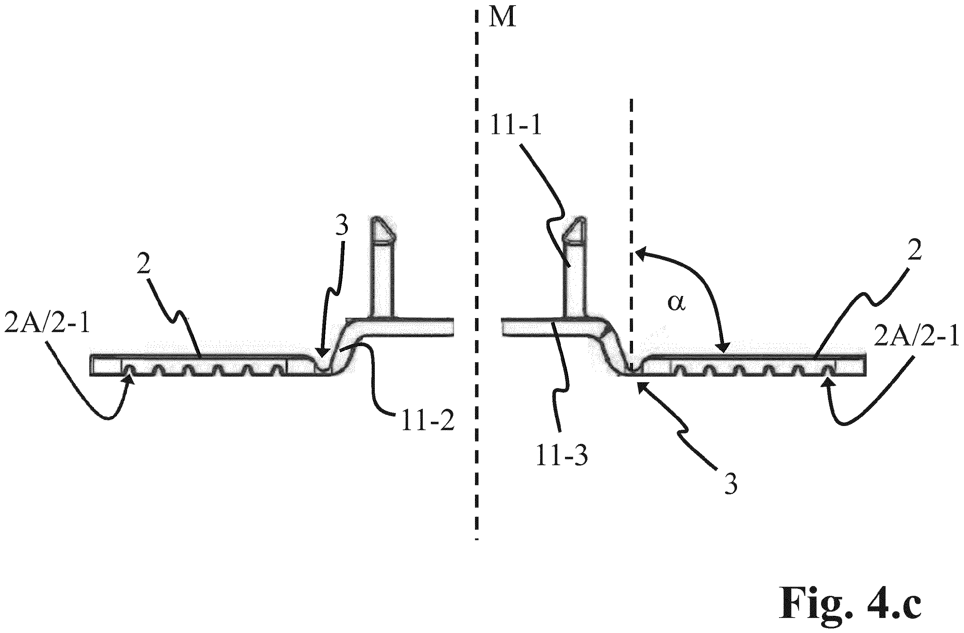

Die Figuren 4.a bis 4.c zeigen eine Detailansicht der beiden Auflagen 2 und des unteren Halterungssegments 11 der Kanülenhalterung 10. Die beiden Auflagen 2 sind über eine Stufe 11-2 mit der unteren Platte 11-3 des Halterungssegments 11 verbunden. Die Stufe 11-2 des unteren Halterungssegments 11 ist zumindest abschnittweise gebogen. Die zwei Auflagen 2 und das untere Halterungssegment 11 sind hier einteilig ausgeführt. Zwischen der einzelnen Auflage 2 und dem unteren Halterungssegment 11 ist jeweils ein Filmscharnier 3 angeordnet oder ausgebildet. Das Filmscharnier 3 wird bereitgestellt durch eine Verdünnung in der Wandstärke. Es wird eine Art Gelenkrille gebildet. Die beiden Flügel 2 sind um eine durch das Folienscharnier 3 gebildete Achse schwenkbar. Die Schwenkachse liegt in Figur 4.c senkrecht zur Blattebene. Die beiden Flügel 2 stellen hier aber keine Flügel zum Greifen des Portkanülensystems 1 dar. Sie stellen vielmehr Elemente zum Höhenausgleich dar. Dadurch kann das Portkanülensystem 1 beispielsweise auf einer nicht ebenen Hautoberfläche sicher positioniert werden. Dass die Flügel 2 keine Flügel zum Herausziehen des Portkanülensystems 1 sind, spiegelt sich in dem Schwenkbereich der beiden Flügel 2 wieder. Die zwei Flügel 2 sind um einen Winkel α von 0° < α ≤ 270° schwenkbar (siehe dazu Figur 4.c). Der Winkel α wird definiert durch den Winkel, welcher zwischen der Längsachse M des Portkanülensystems 1 und der Auflage 2 eingeschlossen wird. Die Längsachse M verläuft im Allgemeinen parallel zur Kanüle 5 und/oder steht senkrecht auf der Ebene, in der die beiden Auflagen 2 im ebenen Zustand liegen. Optional können in der Unterseite 2A der beiden Auflagen 2 eine Vielzahl an, vorzugsweise parallelen, Rillen 2-1 oder Aussparungen eingebracht sein (siehe die Figuren 4.b und 4.c).

Das erfindungsgemäße Portkanülensystem 1 bietet Sicherheitsfunktionen gegen Nadelstichverletzungen, Kostenvorteile, Anwendervorteile zur Beobachtung der Einstichstelle sowie deren verbesserte Versorgung, Patientenvorteile bezüglich Bewegungsfreiheit durch ein komfortables Tragen des Portkanülensystems 1, insbesondere gegenüber Systemen, die zwar die gleichen Baugrößenvorteile, jedoch keine beweglichen Befestigungselemente, wie ein Scharnier, besitzen. Das Portkanülensystem 1 kann zum Beispiel mittels Klebestreifen auf der Haut eines Patienten fixiert werden. Abschließend zeigen die Figuren 5.a und 5.b noch zwei weitere Ausgestaltungen der unteren Kanülenhalterung 11. Als Alternative oder Ergänzung kann auf der Unterseite 2A der Auflage 2 eine Klebeschicht 2-3 vorgesehen sein (Figur 5.a). Dadurch kann das Portkanülensystem 1 direkt auf der Haut eines Patienten fixiert oder zumindest vorfixiert werden. Figur 5.b zeigt eine weitere Ausgestaltung der unteren Kanülenhalterung 10 bzw. der Auflage 2, bei der auf der Unterseite 2A zunächst eine Polsterung 2-2 und erst dann eine Klebeschicht 2-3 aufgebracht ist. Dadurch kann der Tragekomfort verbessert werden.

Es ist dem Fachmann ersichtlich, dass die beschriebenen Ausführungsformen beispielhaft zu verstehen sind. Die Erfindung ist nicht auf diese beschränkt sondern kann in vielfältiger Weise variiert werden, ohne das Wesen der Erfindung zu verlassen, welches durch die Ansprüche definiert wird. Merkmale einzelner Ausführungsformen und die im allgemeinen Teil der Beschreibung genannten Merkmale können, im Rahmen der folgenden Ansprüche, jeweils untereinander als auch miteinander kombiniert werden.

### Bezugszeichenliste:

- 1: Portkanülensystem
- M: Längsachse des Portkanülensystems
- 2: Auflage
- 2A: Unterseite der Auflage
- 2-1: Rillen in der Unterseite der Auflage
- 2-2: Polster oder Auflagepolster
- 2-3: Klebeschicht
- 3: Scharnier oder Folienscharnier
- 4: Zuführung
- 5: Kanüle
- 10: Kanülenhalterung
- 11: Unteres Halterungssegment
- 11-1: Teleskopsegment des unteren Halterungssegments
- 11-2: Stufe des unteren Halterungssegments
- 11-3: Untere Platte des unteren Halterungssegments
- 11-4: Klipslaschen
- 12: Erstes mittleres Halterungssegment oder Teleskopsegment
- 13: Zweites mittleres Halterungssegment oder Teleskopsegment
- 14: Oberes Halterungssegment oder oberes Teleskopsegment
- 15: Griffstück
- 15-1: Gleitrippen am Griffstück
- 16: Bodenplatte

## Patentansprüche

1. Portkanülensystem (1) umfassend eine Kanülenhalterung (10), eine an der Kanülenhalterung (10) befestigte Kanüle (5) mit einer Kanülenspitze, welche wenigstens In eine Punktionsstellung und eine Sicherungsstellung überführbar ist, einen Sicherungsabschnitt (11-1, 12, 13, 14), der die Kanülenspitze In der Sicherungsstellung zumindest abschnittsweise umgibt, eine an der Kanüle (5) angeschlossene Zuführung (4) sowie wenigstens eine mit der Kanülenhalterung (10) verbundene Auflage (2) zum Auflegen des Portkanülensystems (1) auf einer Haut eines Patienten, wobei die wenigstens eine Auflage (2) mittels eines Scharniers (3) mit der Kanülenhalterung (10) verbunden ist, und wobei die Kanülenhalterung (10) ein mit der Auflage (2) verbundenes unteres Halterungssegment (11) umfasst, welches eine Stufe (11-2) bereitstellt, so dass das untere Halterungssegment (11) In einem auf der Haut des Patienten aufgelegten Zustand des Portkanülensystems (1) von der Haut des Patienten beabstandet ist.

2. Portkanülensystem (1) nach Anspruch 1, **dadurch gekennzeichnet, dass** die Auflage (2) um eine durch das Scharnier (3) bereitgestellte Achse In einem Winkelbereich von 0° < α ≤ 270° relativ zu einer Längsachse (M) des Portkanülensystems (1) schwenkbar Ist.

3. Portkanülensystem (1) nach einem der vorstehenden Ansprüche, **dadurch gekennzeichnet, dass** das Scharnier (3) ein Filmscharnier ist.

4. Portkanülensystem (1) nach einem der vorstehenden Ansprüche, **dadurch gekennzeichnet, dass** die Auflage (2) durch wenigstens zwei Platten bereitgestellt wird, welche sich seitlich von der Kanülenhalterung (10) nach außen erstrecken und vorzugsweise gegenüberliegend angeordnet sind.

5. Portkanülensystem (1) nach einem der vorstehenden Ansprüche, **dadurch gekennzeichnet, dass** das Scharnier (3) die Stufe (11-2) des unteren Halterungssegments (11) mit der Auflage (2) verbindet.

6. Portkanülensystem (1) nach einem der vorstehenden Ansprüche, **dadurch gekennzeichnet, dass** die Auflage (2) und/oder die Kanülenhalterung (10) mittels Spritzguss gefertigt sind.

7. Portkanülensystem (1) nach einem der vorstehenden Ansprüche, **dadurch gekennzeichnet, dass** an einer Unterseite (2A) der Auflage (2) zumindest abschnittsweise eine Vielzahl an Rillen (2-1) eingebracht ist.

8. Portkanülensystem (1) nach einem der vorstehenden Ansprüche, **dadurch gekennzeichnet, dass** an einer Unterseite (2A) der Auflage (2) zumindest abschnittsweise eine Polsterung (2-2) angeordnet ist.

9. Portkanülensystem (1) nach einem der vorstehenden Ansprüche, **dadurch gekennzeichnet, dass** eine Unterselte (2A) der Auflage (2) und/oder einer Polsterung (2-2) zumindest abschnittsweise mit einer Schicht (2-3) eines Klebstoffs zum lösbaren Verbinden der Auflage (2) mit der Haut des Patienten bedeckt ist.

10. Portkanülensystem (1) nach einem der vorstehenden Ansprüche, **dadurch gekennzeichnet, dass** die Zuführung (4) an der Kanülenhalterung (10) befestigt ist.

11. Portkanülensystem (1) nach einem der vorstehenden Ansprüche, **dadurch gekennzeichnet, dass** die Kanülenhalterung (10) wenigstens ein mit dem unteren Halterungssegment (11) verbundenes mittleres Halterungssegment (12, 13) und ein mit dem mittleren Halterungssegment (12, 13) oberes Halterungssegment (14) aufweist.

12. Portkanülensystem (1) nach einem der vorstehenden Ansprüche, **dadurch gekennzeichnet, dass** die Kanülenhalterung (10) ein Griffstück (15) umfasst, das vorzugswelse mit dem oberen Halterungssegment (14) und/oder der Zuführung (4) verbunden Ist.

13. Portkanülensystem (1) nach einem der vorstehenden Ansprüche, **dadurch gekennzeichnet, dass** das obere Halterungssegment (14), das wenigstens eine mittlere Halterungssegment (12, 13) und/oder das untere Halterungssegment (11) Teleskopsegmente sind, die. gegeneinander beweglich und untereinander geführt ausgebildet sind, wobei die Teleskopsegmente in der Punktionsstellung zumindest abschnittsweise ineinander geschoben und in der zurückgezogenen Schutzstellung auseinander gezogen sind.

14. Set umfassend ein Portkanülensystem (1) nach einem der vorstehenden Ansprüche, einen Klebestreifen zum Befestigen des Portkanülensystems (1) auf der Haut eines Patienten, eine Kompresse zum Positionieren zwischen dem Portkanülensystem (1) und der Haut eines Patienten, eine auf die Kanüle (5) aufsetzbare oder aufgesetzte Nadelschutzkappe, einen mit der Zuführung (4) verbundenen oder verbindbaren Schlauch, eine Schlauchklemme und/oder einen mit dem Schlauch verbundenen oder verbindbaren Konnektor.

## Claims

1. A port cannula system (1), comprising a cannula holder (10), a cannula (5) secured to the cannula holder (10) with a cannula tip, which can be moved at least into a puncture position and a safety position, a safety section (11-1, 12, 13, 14), which surrounds the cannula tip at least in sections in the safety position, a feeder (4) connected to the cannula (5) and at least one support piece (2) connected to the cannula holder (10) for supporting the port cannula system (1) on a patient's skin, wherein the at least one support piece (2) is connected to the cannula holder (10) by means of a hinge (3) and wherein the cannula holder (10) comprises a lower holder segment (11) connected to the support piece (2) which provides a step (11-2) such that the lower holder segment (11) is spaced apart from the patient's skin in a state of the port cannula system (1) where it is supported on the patient's skin.

2. The port cannula system (1) according to claim 1, **characterized in that** the support piece (2) is pivotable around an axis provided by the hinge (3) in an angular range of 0° < α ≤ 270° relative to a longitudinal axis (M) of the port cannula system (1).

3. The port cannula system (1) according to one of the preceding claims, **characterized in that** the hinge (3) is a film hinge.

4. The port cannula system (1) according to one of the preceding claims, **characterized in that** the support piece (2) is provided by at least two plates which extend laterally outwards from the cannula holder (10) and are preferably arranged opposite one another.

5. The port cannula system (1) according to one of the preceding claims, **characterized in that** the hinge (3) connects the step (11-2) of the lower holder segment (11) to the support piece (2).

6. The port cannula system (1) according to one of the preceding claims, **characterized in that** the support piece (2) and/or the cannula holder (10) are produced by means of injection molding.

7. The port cannula system (1) according to one of the preceding claims, **characterized in that** a plurality of grooves (2-1) is incorporated at least in sections on an underside (2A) of the support piece (2).

8. The port cannula system (1) according to one of the preceding claims, **characterized in that** padding (2-2) is arranged at least in sections on an underside (2A) of the support piece (2).

9. The port cannula system (1) according to one of the preceding claims, **characterized in that** an underside (2A) of the support piece (2) and/or padding (2-2) is covered at least in sections with a layer (2-3) of an adhesive to detachably connect the support piece (2) to the patient's skin.

10. The port cannula system (1) according to one of the preceding claims, **characterized in that** the feeder (4) is secured to the cannula holder (10).

11. The port cannula system (1) according to one of the preceding claims, **characterized in that** the cannula holder (10) has at least one middle holder segment (12, 13) connected to the lower holder segment (11) and an upper holder segment (14) to the middle holder segment (12, 13).

12. The port cannula system (1) according to one of the preceding claims, **characterized in that** the cannula holder (10) comprises a gripping piece (15), which is preferably connected to the upper holder segment (14) and/or to the feeder (4).

13. The port cannula system (1) according to one of the preceding claims, **characterized in that** the upper holder segment (14), the at least one middle holder segment (12, 13) and/or the lower holder segment (11) are telescopic segments, which are formed so as to be movable relative to one another and guided with respect to one another, wherein the telescopic segments are pushed at least in sections into one another in the puncture position and are drawn apart from one another in the retracted protection position.

14. A kit comprising a port cannula system (1) according to one of the preceding claims, an adhesive strip to secure the port cannula system (1) on a patient's skin, a compress to be positioned between the port cannula system (1) and a patient's skin, a needle protection cap that can be placed or is placed on the cannula (5), a hose that is connected or can be connected to the feeder (4), a hose clamp and/or a connector that is connected or can be connected to the hose.

## Revendications

1. Système de canule à orifice (1), comprenant un porte-canule (10), une canule (5) fixée au porte-canule (10) avec une pointe de canule, qui peut être déplacé au moins dans une position de perforation et une position de sécurité, une section de sécurité (11-1, 12, 13, 14), qui entoure la pointe de canule au moins par sections dans la position de sécurité, un dispositif d'alimentation (4) raccordé à la canule (5) et au moins une pièce de support (2) raccordée au porte-canule (10) pour supporter le système de canule à orifice (1) sur la peau d'un patient, dans lequel l'au moins une pièce de support (2) est raccordée au porte-canule (10) au moyen d'une charnière (3) et dans lequel le porte-canule (10) comprend un segment porteur inférieur (11) raccordé à la pièce de support (2) qui fournit une marche (11-2) de sorte que le segment porteur inférieur (11) est espacé de la peau du patient dans un état du système de canule à orifice (1) où il est supporté sur la peau du patient.

2. Système de canule à orifice (1) selon la revendication 1, **caractérisé en ce que** la pièce de support (2) peut pivoter autour d'un axe fourni par la charnière (3) dans une plage angulaire de 0° < α ≤ 270° par rapport à un axe longitudinal (M) du système de canule à orifice (1).

3. Système de canule à orifice (1) selon l'une des revendications précédentes, **caractérisé en ce que** la charnière (3) est une charnière pelliculaire.

4. Système de canule à orifice (1) selon l'une des revendications précédentes, **caractérisé en ce que** la pièce de support (2) est pourvue d'au moins deux plaques qui s'étendent latéralement vers l'extérieur depuis le porte-canule (10) et sont de préférence disposées en face l'une de l'autre.

5. Système de canule à orifice (1) selon l'une des revendications précédentes, **caractérisé en ce que** la charnière (3) raccorde la marche (11-2) du segment porteur inférieur (11) à la pièce de support (2).

6. Système de canule à orifice (1) selon l'une des revendications précédentes, **caractérisé en ce que** la pièce de support (2) et/ou le porte-canule (10) sont produits par moulage par injection.

7. Système de canule à orifice (1) selon l'une des revendications précédentes, **caractérisé en ce qu'**une pluralité de rainures (2-1) est incorporée au moins en sections sur une face inférieure (2A) de la pièce de support (2).

8. Système de canule à orifice (1) selon l'une des revendications précédentes, **caractérisé en ce que** le rembourrage (2-2) est disposé au moins en sections sur une face inférieure (2A) de la pièce de support (2).

9. Système de canule à orifice (1) selon l'une des revendications précédentes, **caractérisé en ce qu'**une face inférieure (2A) de la pièce de support (2) et/ou de rembourrage (2-2) est recouverte au moins en sections d'une couche (2-3) d'un adhésif pour raccorder de manière détachable la pièce de support (2) à la peau du patient.

10. Système de canule à orifice (1) selon l'une des revendications précédentes, **caractérisé en ce que** le dispositif d'alimentation (4) est fixé au support de canule (10).

11. Système de canule à orifice (1) selon l'une des revendications précédentes, **caractérisé en ce que** le porte-canule (10) a au moins un segment porteur central (12, 13) raccordé au segment porteur inférieur (11) et un segment porteur supérieur (14) au segment porteur central (12, 13).

12. Système de canule à orifice (1) selon l'une des revendications précédentes, **caractérisé en ce que** le porte-canule (10) comprend une pièce de préhension (15), qui est de préférence raccordé au segment porteur supérieur (14) et/ou au dispositif d'alimentation (4).

13. Système de canule à orifice (1) selon l'une des revendications précédentes, **caractérisé en ce que** le segment porteur supérieur (14), l'au moins un segment porteur médian (12, 13) et/ou le segment porteur inférieur (11) sont des segments télescopiques, qui sont formés afin d'être mobiles les uns relativement aux autres et guidés les uns par rapport aux autres, dans lequel les segments télescopiques sont poussés au moins par sections les uns dans les autres dans la position de perforation et sont éloignés les uns des autres dans la position de protection rétractée.

14. Kit comprenant un système de canule à orifice (1) selon l'une des revendications précédentes, une bande adhésive pour fixer le système de canule à orifice (1) sur la peau d'un patient, une compresse à positionner entre le système de canule à orifice (1) et la peau d'un patient, un capuchon de protection d'aiguille qui peut être placé ou est placé sur la canule (5), un tuyau qui est raccordé ou peut être raccordé au dispositif d'alimentation (4), un collier de serrage et/ou un raccord qui est raccordé ou peut être raccordé au tuyau.
